# EUROPÄISCHE PATENTANMELDUNG

(11) **EP 2 868 307 A1**
(43) Veröffentlichungstag der Anmeldung: **06.05.2015**
(21) Anmeldenummer: 14188598.8
(22) Anmeldetag: 13.10.2014
(51) Int. Cl.: A61J 7/00, A61M 5/315, G01F 11/02

(54) **Dosiersystem**

(30) Priorität: 31.10.2013 DE 102013222187
(71) Anmelder: RAUMEDIC AG, 95213 Münchberg (DE)
(72) Erfinder: Jakob, Thomas, 95111 Rehau (DE); Skaper, Frank, 95191 Leupoldsgrün (DE); Eichelkraut, Gero, 01279 Dresden (DE); Braun, Thomas, 95032 Hof (DE); Spindler, Bernd, 95233 Helmbrechts (DE)
(74) Vertreter: Rau, Schneck & Hübner Patentanwälte Rechtsanwälte PartGmbB

(57) **Zusammenfassung**

Die Erfindung betrifft ein Dosiersystem zur Einstellung eines Dosiervolumens eines fließfähigen Mediums. Das Dosiersystem umfasst eine Längsachse (1), einen Dosierbehälter (3) zur Aufnahme des Mediums und einen Dosier-Kolbenkörper (4), der in dem Dosierbehälter (3) abgedichtet verlagerbar ist zwischen einer Ausgangsposition und einer Dosierposition. In der Ausgangsposition ist der Dosier-Kolbenkörper (4) ganz in den Dosierbehälter (3) eingeschoben, während dieser in der Dosierposition bis zu einer der gewünschten Dosiermenge des Mediums entsprechenden Wegstrecken aus dem Dosierbehälter (3) herausgezogen ist. Ferner hat das Dosiersystem ein Aufzieh-Begrenzungselement (5) zum Begrenzen eines Aufziehwegs des Dosier-Kolbenkörpers (4) in der Dosierposition, das in einer von mehreren Begrenzungspositionen relativ zu der Längsachse (1) an dem Dosierbehälter (3) axial festlegbar ist. Das Dosiersystem hat außerdem einen Anschlag (6) zur Anlage an dem Aufzieh-Begrenzungselement (5) zum Begrenzen des Aufziehwegs des Dosier-Kolbenkörpers (4) in der Dosierposition. Der Anschlag (6) ist axial fest mit dem Dosier-Kolbenkörper (4) verbunden.

## Beschreibung

Die Erfindung betrifft ein Dosiersystem zur Einstellung eines Dosiervolumens eines fließfähigen Mediums, wie eines flüssigen Medikaments.

Aus dem Stand der Technik sind durch offenkundige Vorbenutzung bereits Dosiersysteme bekannt, deren Dosiervolumen einstellbar sind. Nachteilig an den bekannten Dosiersystemen ist oftmals, dass deren Handhabung nicht bedienerfreundlich ist. Ferner ist die Handhabung nicht immer intuitiv. Im Allgemeinen sind auch nur wenige Dosiervolumen einstellbar, wobei häufig die letztendlich erzielte Dosiermenge stark von der gewünschten Dosiermenge abweicht. Außerdem sind die bekannten Dosiersysteme aufwändig in der Herstellung bzw. Montage.

Der Erfindung liegt daher die Aufgabe zugrunde, ein Dosiersystem zur Einstellung eines Dosiervolumens eines fließfähigen Mediums bereitzustellen, das äußerst bedienerfreundlich und intuitiv ist. Ferner sollen mit dem Dosiersystem möglichst viele Dosiermengen äußerst genau erreichbar und individuell einstellbar sein.

Diese Aufgabe wird erfindungsgemäß durch die in dem unabhängigen Anspruch 1 angegebenen Merkmale gelöst. Der Kern der Erfindung liegt darin, dass durch das in einer Begrenzungsposition an dem Dosierbehälter axial festgelegte Aufzieh-Begrenzungselement der Aufziehweg des Dosier-Kolbenkörpers begrenzbar ist. An dem Dosier-Kolbenkörper ist dafür ein entsprechender, axial fester Anschlag vorgesehen. Die maximale Dosiermenge ist in Abhängigkeit der axialen Festlegung des Aufzieh-Begrenzungselements an dem Dosierbehälter einstellbar.

Das Aufzieh-Begrenzungselement bildet einen Gegenanschlag für den Anschlag. Durch diesen taktilen bzw. mechanischen Anschlag ist im Gegensatz zu rein visuellen Einstellungen eine Fehldosierung unmöglich. Bei herkömmlichen Dosierspritzen liegen fehlerhafte Dosierungen durchschnittlich bei ca. 35 % bezogen auf die Dosiermenge, während bei üblichen Dosierlöffeln fehlerhafte Dosierungen sogar durchschnittlich bei ca. 85 % bezogen auf die Dosiermenge liegen. Der taktile bzw. mechanische Anschlag führt also zu einer erhöhten Sicherheit, was insbesondere bei hochaktiven Medikamenten und geringen Dosiervolumina vorteilhaft ist. Auch visuell beeinträchtigte Menschen können so eine genaue Dosierung einstellen bzw. vornehmen.

Das Aufzieh-Begrenzungselement ist radial verlagerbar. Es ist von Vorteil, wenn das Aufzieh-Begrenzungselement senkrecht zu der Längsachse zwischen seinen radialen Positionen verlagerbar ist. Eine schräge Verlagerung gegenüber der Längsachse ist alternativ möglich.

Das Aufzieh-Begrenzungselement ist nur imstande, an dem Anschlag anzuliegen bzw. mit diesem wechselzuwirken, wenn es sich in seiner radial inneren Position befindet. Umgekehrt bedeutet dies, dass das Aufzieh-Begrenzungselement in seiner radial äußeren Position unwirksam ist bzw. außer Stande ist, mit dem Anschlag wechselzuwirken.

Der Dosier-Kolbenkörper ist bis zu der Anlage zwischen dem Aufzieh-Begrenzungselement und dem Anschlag vorzugsweise frei verschiebbar.

Günstigerweise sind die Begrenzungspositionen durch erste Formschlussmittel an dem Dosierbehälter vorgegeben. Es ist von Vorteil, wenn an dem Aufzieh-Begrenzungselement mindestens ein zweites Formschlussmittel zum Wechselwirken mit mindestens einem der ersten Formschlussmittel angeordnet ist. Alternativ ist das Aufzieh-Begrenzungselement stufenlos an dem Dosierbehälter axial festlegbar, sodass quasi keine genauen Begrenzungspositionen vorgegeben sind. Es ist von Vorteil, wenn die Verbindung zwischen mindestens einem ersten Formschlussmittel an dem Dosierbehälter und mindestens einem zweiten Formschlussmittel an dem Aufzieh-Begrenzungselement eine Steckverbindung bzw. Rastverbindung ist.

Vorteilhafterweise kommt das Dosiersystem in der Kindermedizin oder bei der Behandlung alter Menschen zum Einsatz.

Die mindestens eine Kommunizierungsöffnung erlaubt ein Eintreten des Mediums in den Dosierbehälter. Ferner ist das in dem Aufnahmeraum befindliche Medium über die mindestens eine Kommunizierungsöffnung wieder aus dem Aufnahmeraum abgebbar.

In der herausgezogenen Stellung des Dosier-Kolbenkörpers ist die Dosiermenge in ihrem Volumen maximal, während sie in der eingeschobenen Stellung des Dosier-Kolbenkörpers dagegen minimal ist.

Das Aufzieh-Begrenzungselement ist vorzugsweise klammerartig ausgeführt. Es ist zweckmäßig, wenn das Aufzieh-Begrenzungselement symmetrisch, bevorzugter U-förmig, ausgeführt ist.

Günstigerweise ist das Dosiersystem spritzenartig ausgeführt. Das Design orientiert sich vorzugsweise an bekannten Dosierspritzen, die Benutzern geläufig sind. Das Dosiersystem ist vorzugsweise äußerst kompakt.

Es ist von Vorteil, wenn das Dosiersystem keine losen Teile hat. Die Teile des Dosiersystems sind dann verliersicher.

Günstigerweise unterscheidet sich das Aufzieh-Begrenzungselement in seiner Farbe von dem Dosierbehälter. Es ist so besonders schnell und sicher optisch wahrnehmbar.

Es ist von Vorteil, wenn der Dosierbehälter aus einem Kunststoffmaterial, vorzugsweise aus einem transparenten Kunststoffmaterial, gebildet ist. Vorzugsweise ist der Dosier-Kolbenkörper aus einem Kunststoffmaterial gebildet. Es ist zweckmäßig, wenn auch das Aufzieh-Begrenzungselement aus einem Kunststoffmaterial gebildet ist.

Weitere vorteilhafte Ausgestaltungen der Erfindung sind in den Unteransprüchen angegeben.

Gemäß dem Unteranspruch 2 ist das Aufzieh-Begrenzungselement außen an dem Dosierbehälter zwischen mindestens zwei Dosierpositionen axial verlagerbar, wenn es sich in seiner radial äußeren Position befindet. Bevorzugter ist das Aufzieh-Begrenzungselement in mindestens fünf, bevorzugter in mindestens zehn, bevorzugter in mindestens 15, verschiedenen, in Richtung der Längsachse voneinander beabstandeten Positionen außen an dem Dosierbehälter axial festlegbar. Vorzugsweise handelt es sich bei den Positionen um vorbestimmte Positionen. Alternativ ist das Aufzieh-Begrenzungselement in Richtung der Längsachse stufenlos verlagerbar bzw. stufenlos außen an dem Dosierbehälter axial festlegbar. Die Dosierpositionen können also vorgegeben oder nicht-vorgegeben, also frei wählbar, sein. Durch diese Ausgestaltung ist eine äußerst feine Dosierinkrementierung mit vielen Dosierinkrementen geschaffen. Günstigerweise ist also eine Außenjustierung bzw. -Einstellung vorhanden.

Das Aufzieh-Begrenzungselement ist außen an dem Dosierbehälter zwischen mindestens zwei Dosierpositionen in Richtung der Längsachse reversibel oder irreversibel axial verlagerbar, wenn es sich in seiner radial äußeren Position befindet.

Es ist von Vorteil, wenn in einer radial äußeren Reinigungsposition des Aufzieh-Begrenzungselements ein Ausziehen des Kolbenkörpers möglich ist.

Die in dem Unteranspruch 3 angegebene Ausgestaltung ermöglicht ein besonders einfaches Auffinden der Begrenzungspositionen. Günstigerweise erfolgt die akustische und/oder taktile Rückmeldung, wenn bei einer Relativbewegung zwischen dem Aufzieh-Begrenzungselement und dem Dosierbehälter die ersten und zweiten Formschlussmittel miteinander wechselwirken. Vorteilhafterweise rattert so das Aufzieh-Begrenzungselement auf dem Dosierbehälter bei einer Relativbewegung zwischen dem Dosierbehälter und dem Aufzieh-Begrenzungselement. Es ist von Vorteil, wenn die ersten Formschlussmittel Formschlussaufnahmen, -öffnungen bzw. - fenster sind, die in Richtung der Längsachse axial beabstandet zueinander an dem Dosierbehälter angeordnet sind.

Die Ausgestaltung gemäß dem Unteranspruch 4 führt zu einer verliersicheren Anordnung des Aufzieh-Begrenzungselements an dem Dosierbehälter. Alternativ ist das Aufzieh-Begrenzungselement von dem Dosierbehälter lösbar. Dies ist insbesondere zu Reinigungszwecken vorteilhaft. Die Rückhalteeinrichtung erschwert bzw. verhindert vorzugsweise eine radiale Bewegung des Aufzieh-Begrenzungselements von der radial inneren Position zu der radial äußeren Position und/oder von der radial äußeren Position weiter nach außen.

Die in dem Unteranspruch 5 angegebene Verbindung ist äußerst sicher und einfach herstellbar. Alternativ ist eine Steckverbindung vorgesehen.

Gemäß dem Unteranspruch 6 umgreift das Aufzieh-Begrenzungselement einen äußeren Umfangsabschnitt des Dosierbehälters. Vorzugsweise gilt dies sowohl für die radial innere als auch die radial äußere Position des Aufzieh-Begrenzungselements.

Durch die Ausgestaltung gemäß dem Unteranspruch 7 ist die einzustellende Dosiermenge äußerst exakt und einfach durch das Aufzieh-Begrenzungselement einstellbar.

Die Ausgestaltung gemäß dem Unteranspruch 8 ermöglicht die Einstellung einer äußerst großen maximalen Dosiermenge.

Die einstückige Ausgestaltung zwischen dem Anschlag und dem Dosier-Kolbenkörper gemäß dem Unteranspruch 9 führt zu einem äußerst funktionssicheren und kostengünstig herstellbaren Dosiersystem.

Die Ausgestaltung gemäß dem Unteranspruch 10 ergibt ein Dosiersystem, das äußerst funktionssicher ist und besonders einfach herstellbar ist.

Die Ausgestaltung gemäß dem Unteranspruch 11 ergibt eine geschützte Anordnung des Dosier-Kolbenauslegers. Ferner ist so dessen Bewegung besonders gut vorgebbar.

Die Ausgestaltung gemäß dem Unteranspruch 13 bewirkt, dass der Aufnahmeraum nicht durch die Führungsaufnahme reduziert bzw. beeinträchtigt ist. Eine Trennung zwischen der Einstellung des Dosiervolumens und der Dosierung des Mediums liegt somit vor.

Nachfolgend werden unter Bezugnahme auf die beigefügte Zeichnung zwei bevorzugte Ausführungsformen der Erfindung beispielhaft beschrieben. Dabei zeigen:

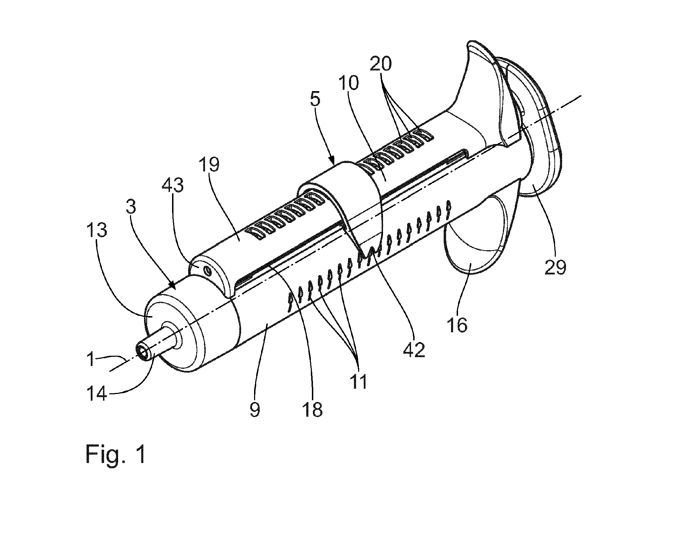

- Fig. 1: eine perspektivische Ansicht eines erfindungsgemäßen Dosiersystems, bei dem sich das Aufzieh-Begrenzungselement in seiner radial inneren Begrenzungsposition befindet,

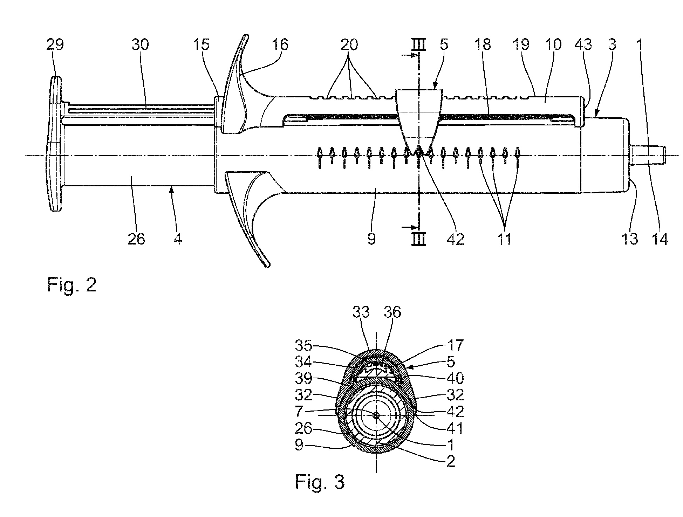

- Fig. 2: eine Seitenansicht des in Fig. 1 dargestellten Dosiersystems,
- Fig. 3: einen Querschnitt durch das Dosiersystem entsprechend der in Fig. 2 gezeigten Schnittlinie III-III,

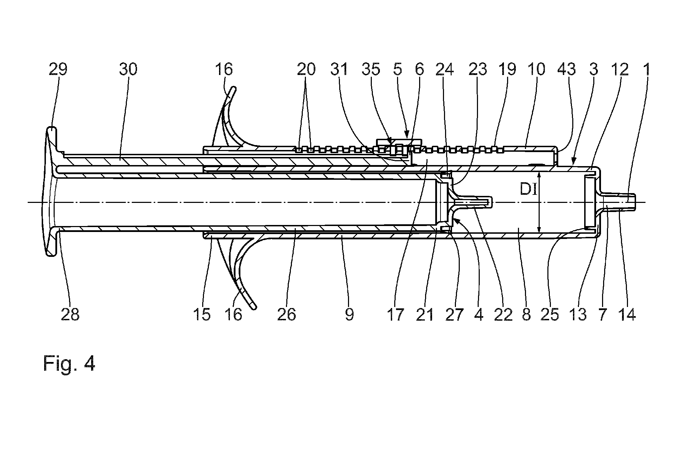

- Fig. 4: einen Längsschnitt durch das in den Fig. 1 und 2 dargestellte Dosiersystem,

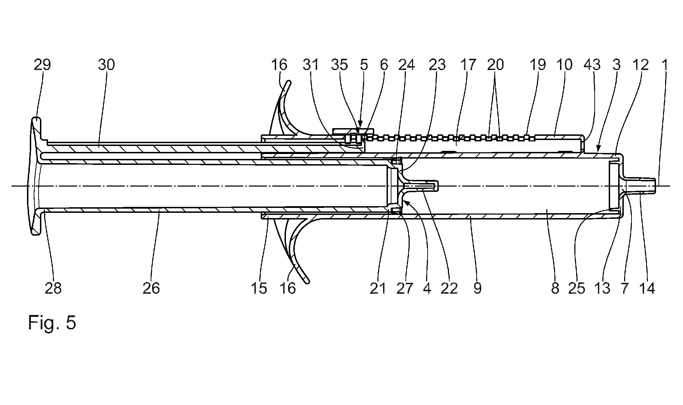

- Fig. 5: einen Längsschnitt entsprechend Fig. 4, wobei sich das Aufzieh-Begrenzungselement in einer axial anderen Dosierposition befindet,
- Fig. 6: eine Seitenansicht entsprechend Fig. 2, wobei sich das Aufzieh-Begrenzungselement in seiner radial äußeren Position befindet,
- Fig. 7: einen Querschnitt entsprechend der in Fig. 6 veranschaulichten Schnittlinie VII-VII,
- Fig. 8: einen Längsschnitt durch das in Fig. 6 dargestellte Dosiersystem,
- Fig. 9: einen Querschnitt entsprechend Fig. 3 durch ein erfindungsgemäßes Dosiersystem gemäß einer zweiten Ausführungsform, bei dem sich das Aufzieh-Begrenzungselement in seiner radial inneren Begrenzungsposition befindet, und
- Fig. 10: einen Querschnitt entsprechend Fig. 6 des in Fig. 9 gezeigten Dosiersystems, bei dem sich das Aufzieh-Begrenzungselement in seiner radial äußeren Position befindet.

Ein in der Zeichnung dargestelltes Dosiersystem hat eine Längsachse 1 und ist bezüglich einer Symmetrieebene 2 symmetrisch ausgeführt. Die Längsachse 1 liegt in der Symmetrieebene 2.

Das Dosiersystem umfasst einen Dosierbehälter 3 und einen in dem Dosierbehälter 3 relativ zu diesem in Richtung der Längsachse 1 verschiebbar geführten Dosier-Kolbenkörper 4. Der Dosierbehälter 3 dient zur Aufnahme eines fließfähigen Mediums. Er begrenzt einen Aufbewahrungsraum 8 für das fließfähige Medium (nicht dargestellt) und hat eine Kommunizierungsöffnung 7 zur Kommunizierung mit der Umgebung. Der Dosier-Kolbenkörper 4 ist in dem Dosierbehälter 3 zwischen einer Ausgangsposition und einer Dosierposition axial gegenüber dem Dosierbehälter 3 verschiebbar. In der Ausgangsposition ist der Dosier-Kolbenkörper 4 ganz in den Dosierbehälter 3 eingeschoben, während dieser in der Dosierposition bis zu einer der gewünschten Dosiermenge des Mediums entsprechenden Wegstrecke aus dem Dosierbehälter 3 herausgezogen ist. Die verwendeten Bezeichnungen "axial" bzw. "radial" beziehen sich jeweils auf die Längsachse 1.

Das Dosiersystem hat außerdem ein Aufzieh-Begrenzungselement 5, das an dem Dosierbehälter 3 in verschiedenen, in Richtung der Längsachse 1 voneinander beabstandeten Begrenzungspositionen axial festlegbar ist und so den maximalen Aufziehweg des Dosier-Kolbenkörpers 4 örtlich begrenzt. An dem Dosier-Kolbenkörper 4 ist zur mechanischen Wechselwirkung mit dem Aufzieh-Begrenzungselement 5 in der Dosierposition ein Anschlag 6 angeordnet. In Abhängigkeit der axialen Anordnung des Aufzieh-Begrenzungselements 5 gegenüber dem Dosierbehälter 3 ist der maximale Aufziehweg des Dosier-Kolbenkörpers 4 bzw. die maximale Dosiermenge individuell einstellbar. Der maximale Aufziehweg ist durch das Aufzieh-Begrenzungselement 5 vorgegeben.

Wenn der Dosier-Kolbenkörper 4 von seiner Ausgangsposition in seine Dosierposition durch Ziehen axial überführt wird, kann ein flüssiges Medium über die Kommunizierungsöffnung 7 in den Aufnahmeraum 8 eingesaugt werden. Wenn dagegen der Dosier-Kolbenkörper 4 von einer Dosierposition in seine Ausgangsposition durch Drücken axial überführt wird, kann dagegen das in dem Aufnahmeraum 8 befindliche Medium über die Kommunizierungsöffnung 7 abgegeben bzw. hinausgespritzt werden.

Der Dosierbehälter 3 umfasst eine sich längs der Längsachse 1 erstreckende Dosierhülse 9 und einen seitlich an die Dosierhülse 9 angesetzten Einstellansatz 10. Die Dosierhülse 9 und der Einstellansatz 10 sind einstückig aus einem Kunststoffmaterial gebildet, bevorzugter gespritzt.

Die Dosierhülse 9 hat eine sich längs der Längsachse 1 erstreckende Dosierskala 11 und ist als Zylinderkörper ausgeführt, der im Wesentlichen einen konstanten Innendurchmesser DI in Richtung der Längsachse 1 hat und an einem ersten Ende 12 durch einen Dosierhülsenboden 13 verschlossen ist. Der Dosierhülsenboden 13 erstreckt sich senkrecht zu der Längsachse 1. In dem Dosierhülsenboden 13 ist mittig die Kommunizierungsöffnung 7 angeordnet. An den Dosierhülsenboden 13 schließt sich im Bereich der Kommunizierungsöffnung 7 außen ein Rohrstück 14 an, dessen Innendurchmesser vorzugsweise wesentlich kleiner als der Innendurchmesser DI der Dosierhülse 9 ist. Die Dosierhülse 9 und der Dosierhülsenboden 13 begrenzen zusammen den Aufnahmeraum 8. Alternativ ist kein Rohrstück 14 vorhanden, sodass die Dosierhülse 9 zum Ausspritzen des fließfähigen Mediums über ihre gesamte Länge im Wesentlichen einen konstanten Durchmesser hat. Alternativ ist anstelle des Rohrstücks 14 ein Luer-Lock-Anschluss vorhanden, um beispielsweise andere Spritzen, Infusionsinstrumente oder dergleichen anschließen zu können.

Die Dosierhülse 9 hat außerdem ein zweites Ende 15, das gegenüberliegend zu dem ersten Ende 12 angeordnet ist. Im Bereich des zweiten Endes 15 springen zwei Griffstücke 16 radial nach außen vor. Die beiden Griffstücke 16 liegen einander gegenüber. Eine andere Anordnung bzw. Ausgestaltung von Griffstücken 16 ist alternativ möglich.

Der Einstellansatz 10 erstreckt sich seitlich an der Dosierhülse 9 längs der Längsachse 1. Er beginnt im Wesentlichen bei dem zweiten Ende 15 und endet benachbart, aber beabstandet zu dem ersten Ende 12. Alternativ erstreckt er sich bis zu dem ersten Ende 12. Der Einstellansatz 10 schließt sich über einen Umfangsbereich von etwa 80° um die Längsmittelachse 1 seitlich an die Dosierhülse 9 an.

Der Einstellansatz 10 ist hohl ausgeführt und begrenzt so eine innere Führungsaufnahme 17 nach außen. Im Querschnitt ist der Einstellansatz 10 bogenförmig ausgebildet. Andere Querschnitte sind alternativ möglich. Der Einstellansatz 10 ist bei dem zweiten Ende 15 axial offen, während dieser an seinem anderen, gegenüberliegenden Ende 43 im Wesentlichen axial geschlossen ist. Alternativ ist der Einstellansatz 10 an dem Ende 43 zumindest bereichsweise offen.

Zwischen der Dosierhülse 9 und dem Einstellansatz 10 erstrecken sich an dem Dosierbehälter 3 längs der Längsachse 1 zwei seitliche Rückhalte-Führungsnuten 18, die auch parallel zueinander verlaufen, einander gegenüberliegend angeordnet sind und in einander gegensinnigen Richtungen nach seitlich außen offen sind. Gemäß einer alternativen Ausführungsform sind mehr als zwei seitliche Rückhalte-Führungsnuten 18 an dem Dosierbehälter 3 vorhanden.

An der der Dosierhülse 9 abgewandten Außenseite 19 des Einstellansatzes 10 sind erste Formschlussmittel 20 vorgesehen, die in einer sich längs der Längsachse 1 erstreckenden Reihe beabstandet zueinander angeordnet sind. Die ersten Formschlussmittel 20 sind jeweils als Öffnungen bzw. Fenster ausgeführt. Sie sind zu der Führungsaufnahme 17 hin offen. Vorzugsweise haben die ersten Formschlussmittel 20 einen rechteckigen Querschnitt und verlaufen parallel zueinander. Andere Querschnitte sind alternativ möglich. Anstelle mehrerer erster Formschlussmittel 20 ist alternativ ein einziges erstes Formschlussmittel vorhanden, das sich längs der Längsachse 1 an der Dosierhülse 9 erstreckt und so längs der Längsachse 1 durchgängig ist, was eine stufenlose Begrenzung des Aufziehwegs des Dosier-Kolbenkörpers 4 erlaubt.

Der Dosier-Kolbenkörper 4 hat an seinem ersten Ende 21 einen in Richtung der Längsachse 1 vorspringenden Verdrängerkörper bzw. Ausstoßkörper 22, dessen Außendurchmesser im Wesentlichen entsprechend dem Innendurchmesser des Rohrstücks 14 ist. Die axiale Länge des Verdrängerkörpers 22 entspricht im Wesentlichen der axialen Länge des Rohrstücks 14. Sie ist vorzugsweise geringfügig größer. Der Verdrängerkörper 22 ist im Wesentlichen zylindrisch ausgeführt und hat einen ringförmigen Querschnitt. Er ist vorne geschlossen. Eine vollzylindrische Ausgestaltung ist alternativ möglich.

An den Verdrängerkörper 22 schließt sich ein radial vorspringender Kolbenboden 23 an, der Bestandteil des Dosier-Kolbenkörpers 4 ist. Der Kolbenboden 23 hat einen Außendurchmesser, der kleiner als der Innendurchmesser DI der Dosierhülse 9 ist, so dass im Bereich des Kolbenbodens 23 ein um die Längsachse 1 laufender Ringspalt 24 vorhanden ist. Der Ringspalt 24 ist nach radial außen durch einen ringförmigen Dichtvorsprung 27 begrenzt, der innenseitig dicht an der Wandung der Dosierhülse 9 anliegt und in einem gewissen Maße flexibel ist.

Von dem Dosierhülsenboden 13 springt in Richtung der Längsachse 1 ein Ringsteg 25 vor, der sich um die Längsachse 1 erstreckt und beabstandet zu der Wandung der Dosierhülse 9 angeordnet ist. Wenn der Dosier-Kolbenkörper 4 vollständig in die Dosierhülse 9 eingeschoben ist, so greift der Ringsteg 25 formschlüssig in den Ringspalt 24 ein, was zu einer besonders guten bzw. vollständigen Restentleerung des Dosiersystems führt.

An den Kolbenboden 23 schließt sich unter Bildung des Ringspalts 24 ein Grundkörper 26 des Dosier-Kolbenkörpers 4 an, der zylindrisch ausgeführt ist. Der Grundkörper 26 hat einen Außendurchmesser, der im Wesentlichen dem Innendurchmesser DI der Dosierhülse 9 entspricht. Der Außendurchmesser des Grundkörpers 26 ist vorzugsweise geringfügig kleiner als der Innendurchmesser DI. Der Ringsteg 25 geht von dem Grundkörper 26 aus.

Gegenüberliegend zu dem ersten Ende 21 hat der Dosier-Kolbenkörper 4 ein zweites Ende 28, an dem sich ein nach seitlich außen vorspringender Betätigungsvorsprung 29 befindet.

Von dem Betätigungsvorsprung 29 springt beabstandet zu dem Grundkörper 26 ein Dosier-Kolbenausleger 30 vor, der sich parallel zu der Längsachse 1 in Richtung auf das Ende 43 des Einstellansatzes 10 erstreckt. Der Dosier-Kolbenausleger 30 hat eine axiale Länge, die im Wesentlichen der axialen Länge des Einstellansatzes 10 entspricht. Der radiale Abstand zwischen dem Grundkörper 26 und dem Dosier-Kolbenausleger 30 entspricht in etwa der Stärke der Wandung der Dosierhülse 9.

Der Dosier-Kolbenausleger 30 hat ein freies axiales Ende 31, an dem der Anschlag 6 angeordnet ist. Der Anschlag 6 springt gegenüber dem Dosier-Kolbenausleger 30 radial nach außen vor. Er ist axial gegenüber dem Grundkörper 26 unbeweglich.

Der Dosier-Kolbenausleger 30 greift über das zweite Ende 15 in die Führungsaufnahme 17 ein und ist in dieser verschiebbar geführt. Durch die Kopplung über den Betätigungsvorsprung 29 bewirkt eine Verlagerung des Grundkörpers 26 auch eine entsprechende gleichsinnige Verlagerung des Dosier-Kolbenauslegers 30, der den Anschlag 6 trägt.

Das Aufzieh-Begrenzungselement 5 ist klammerartig ausgeführt und hat zwei einander gegenüberliegende U-Schenkel 32, die über einen äußeren U-Boden 33 miteinander verbunden sind. Die U-Schenkel 32 sind in einem gewissen Maße federnd zueinander auslenkbar.

Der U-Boden 33 hat eine dem Einstellansatz 10 zugewandte Innenseite 34, deren Krümmung im Wesentlichen an die Außenkrümmung des Einstellansatzes 10 angepasst ist. Von der Innenseite 34 des U-Bodens 33 springen zwei zweite Formschlussmittel 35 in Richtung auf die Längsachse 1 vor. Die zweiten Formschlussmittel 35 sind axial in Richtung der Längsachse 1 beabstandet zueinander angeordnet. Die zweiten Formschlussmittel 35 sind derart angeordnet und ausgebildet, dass diese in zwei benachbart zueinander angeordnete erste Formschlussmittel 20 einrastbar sind, was dann zu einer Fixierung des Aufzieh-Begrenzungselements 5 gegenüber dem Einstellansatz 10 und somit gegenüber dem Dosierbehälter 3 führt. Jedes zweite Formschlussmittel 35 umfasst zwei zweite Formschlussvorsprünge 36. Jeder zweite Formschlussvorsprung 36 hat eine nach seitlich außen vorspringende Rastnase 37, die wiederum eine der Innenseite 34 des U-Bodens 33 zugewandte Rückhaltefläche 38 hat. Alternativ ist genau ein zweites Formschlussmittel 35 vorhanden.

Von jedem U-Schenkel 32 wiederum springt eine Rückhaltenase 39 innenseitig vor, die eine der Innenseite 34 des U-Bodens 33 zugewandte zweite Rückhaltefläche 40 hat. Die beiden Rückhaltenasen 39 springen von den U-Schenkeln 32 aufeinander zu und sind jeweils zwischen dem U-Boden 33 und freien Enden 41 der U-Schenkel 32 angeordnet. Jeder U-Schenkel 32 hat zwischen seiner Rückhaltenase 39 und seinem freien Ende 41 jeweils eine Krümmung, die an die äußere Krümmung der Dosierhülse 9 angepasst ist.

Im Bereich der freien Enden 41 ist bei jedem U-Schenkel 32 eine Markierung 42 vorgesehen, die hier als V-förmige Vertiefung ausgeführt ist. Andere Ausgestaltungen der Markierung 42 sind alternativ möglich.

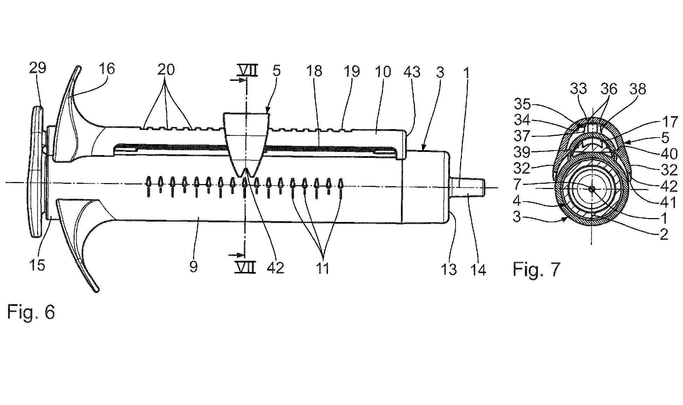

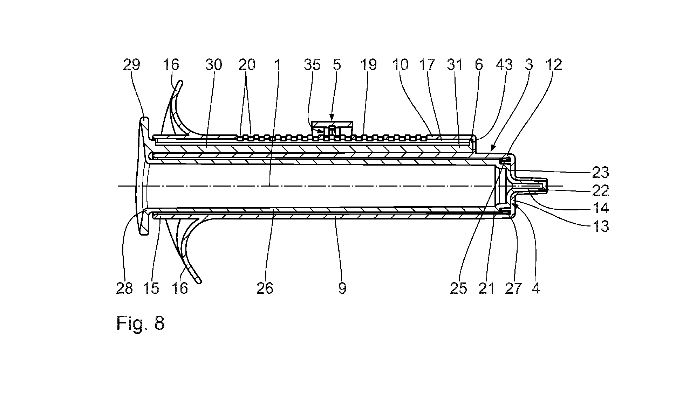

Nachfolgend wird das Dosiersystem im Einsatz näher beschrieben. Dabei wird zunächst von einem Zustand ausgegangen, bei dem sich das Aufzieh-Begrenzungselement 5 in seiner radial äußeren, unwirksamen Position befindet. Dieser Zustand ist in den Fig. 6 bis 8 veranschaulicht.

Bei diesem Zustand stehen die zweiten Formschlussmittel 35 außer Eingriff mit den ersten Formschlussmitteln 20, so dass das Aufzieh-Begrenzungselement 5 längs der Längsachse 1 gegenüber der Dosierhülse 9 bzw. dem Dosierbehälter 3 verschiebbar ist. Die axiale Verschiebung des Aufzieh-Begrenzungselements 5 gegenüber der Dosierhülse 9 ist durch die Rückhaltenasen 39 geführt, die von außen seitlich in die Rückhalte-Führungsnuten 18 eingreifen. Durch den Eingriff der Rückhaltenasen 39 in die Führungsnuten 18 wird eine Entfernung des Aufzieh-Begrenzungselements 5 nach radial außen erschwert bzw. verhindert.

Die an dem Einstellansatz 10 im Bereich der Rückhalte-Führungsnuten 18 angreifenden Rückhalteflächen 40 verhindern bzw. erschweren dabei ein Entfernen des Aufzieh-Begrenzungselements 5 in radialer Richtung von dem Dosierbehälter 3.

Beim Verschieben des Aufzieh-Begrenzungselements 5 greifen die zweiten Formschlussmittel 35 kurzfristig etwas in die ersten Formschlussmittel 20 ein, was zu einer akustischen Rückmeldung in Form eines Rattergeräusches führt.

Das Aufzieh-Begrenzungselement 5 wird dann in einer Begrenzungsposition axial an dem Einstellansatz 10 festgelegt. Die Begrenzungsposition gibt dann eine gewünschte Dosiermenge des Mediums vor. Das Einstellen der gewünschten Dosiermenge wird durch die an dem Dosierbehälter 3 vorgesehene Dosierskala 11 und die Markierung 42 vereinfacht. Die Markierung 42 zeigt die jeweilige Dosiermenge an, die aufgrund der Einstellung des Aufzieh-Begrenzungselements 5 maximal in dem Aufnahmeraum 8 aufnehmbar ist.

Das axiale Festlegen des Aufzieh-Begrenzungselements 5 gegenüber dem Dosierbehälter 3 wird erreicht, indem auf den U-Boden 33 derart von außen eine manuelle Kraft in Richtung auf die Längsachse 1 ausgeübt wird, dass sich der U-Boden 33 der Außenseite 19 des Einstellansatzes 10 nähert, wobei die zweiten Formschlussmittel 35 in die entsprechenden benachbarten ersten Formschlussmittel 20 eingreifen. Die radial innere Position des Aufzieh-Begrenzungselements 5 ist erreicht, wenn die Innenseite 34 des U-Bodens 33 an der Außenseite 19 des Einstellansatzes 10 zumindest bereichsweise anliegt bzw. unmittelbar benachbart zu dieser angeordnet ist und so die zweiten Formschlussmittel 35 mit den entsprechenden benachbarten ersten Formschlussmitteln 20 rastend in Eingriff stehen.

Die zweiten Formschlussmittel 35 greifen von außen durch die ersten Formschlussmittel 20 in die Führungsaufnahme 17 ein und stehen nach innen gegenüber den ersten Formschlussmitteln 20 über. Die ersten Rückhalte flächen 38 hintergreifen die die entsprechenden ersten Formschlussmittel 20 begrenzende Wandung des Einstellansatzes 10 und verhindern bzw. erschweren so ein Rückführen des Aufzieh-Begrenzungselements 5 in seine radial äußere Position.

Die U-Schenkel 32 liegen im Bereich zwischen den freien Enden 41 und den Rückhaltenasen 39 außen flächig an der Dosierhülse 9 an. Die Rückhaltenasen 39 sind von den Rückhalte-Führungsnuten 18 nun entfernt.

Das Aufzieh-Begrenzungselement 5 ist nun axial und radial sowie in Umfangsrichtung um die Längsachse 1 an dem Dosierbehälter 3 fixiert. Es liegt eine Außenverrastung vor.

Das dem axialen Ende 43 des Einstellansatzes 10 zugewandte zweite Formschlussmittel 35 ragt in die Führungsaufnahme 17 und bildet dabei einen Gegenanschlag für den Anschlag 6, der sich ebenfalls in der Führungsaufnahme 17 befindet. Der Aufziehweg des Dosier-Kolbenkörpers 4 ist so begrenzt. In der Dosierposition liegt der Anschlag 6 an der dem Ende 43 des Einstellansatzes 10 zugewandten Seite an dem Gegenanschlag an und verhindert so eine weitere Ausziehbewegung des Dosier-Kolbenkörpers 4.

Die Rastnasen 37 und die Rückhaltenasen sind Bestandteil einer Rückhalteeinrichtung.

Die axiale Verlagerung des Dosier-Kolbenkörpers 4 wird durch Benutzung der Griffstücke 16 und des Betätigungsvorsprungs 29 vereinfacht, die die Aufbringung entsprechender Verlagerungskräfte erlauben.

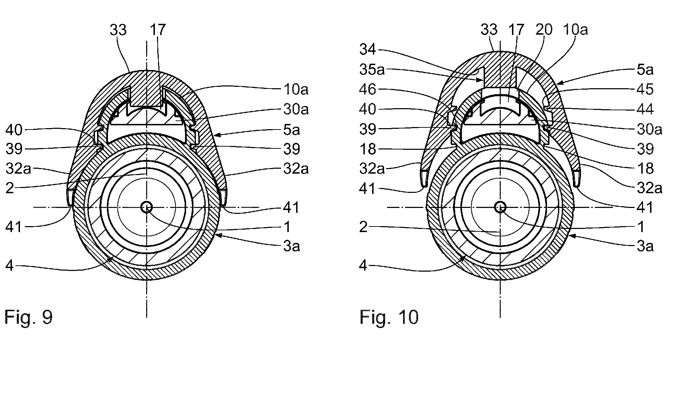

Nachfolgend wird unter Bezugnahme auf die Fig. 9 und 10 eine zweite Ausführungsform beschrieben. Konstruktiv identische Teile erhalten dieselben Bezugszeichen wie bei der vorherigen Ausführungsform, auf die hiermit verwiesen wird. Konstruktiv unterschiedliche, jedoch funktionell gleichartige Teile erhalten dieselben Bezugszeichen wie bei der vorherigen Ausführungsform mit einem nachgeordneten "a".

Bei dieser Ausführungsform sind der Dosierbehälter 3a und das Aufzieh-Begrenzungselement 5a konstruktiv anders als bei der vorherigen Ausführungsform ausgeführt.

Das Aufzieh-Begrenzungselement 5a hat im Gegensatz zu der vorherigen Ausführungsform zusätzlich zu den Rückhaltenasen 39 noch zwei zweite Rückhaltenasen 44. Von jedem U-Schenkel 32a springt eine der zweiten Rückhaltenasen 44 innenseitig vor, die wiederum eine der Innenseite 34 des U-Bodens 33 zugewandte Rückhaltefläche 45 hat. Die beiden zweiten Rückhaltenasen 44 springen von den U-Schenkeln 32a aufeinander zu und sind jeweils zwischen dem U-Boden 33 und den Rückhaltenasen 39 angeordnet, die somit erste Rückhaltenasen 39 bilden. Die zweiten Rückhaltenasen 44 sind benachbart zu den ersten Rückhaltenasen 39 angeordnet und verlaufen parallel zu diesen. Sie sind näher an den ersten Rückhaltenasen 39 als an dem U-Boden 33 angeordnet. Die zweiten Rückhaltenasen 44 sind im Wesentlichen in ihrer Form identisch zu den ersten Rückhaltenasen 39 ausgeführt.

Von der Innenseite 34 des U-Bodens 33 springt ein zweites Formschlussmittel 35a in Richtung auf die Längsachse 1 vor. Im Gegensatz zu der vorherigen Ausführungsform ist hier das zweite Formschlussmittel 35a als Steckvorsprung ausgeführt. Das zweite Formschlussmittel 35a ist plattenförmig oder blockförmig ausgeführt. Es ist frei von Rastnasen oder dergleichen. Alternativ ist mehr als genau ein zweites Formschlussmittel 35a vorhanden.

Verglichen mit der vorherigen Ausführungsform verlaufen an dem Einstellansatz 10a des Dosierbehälters 3a längs der Längsachse 1 zusätzlich zu den Rückhalte-Führungsnuten 18 zwei seitliche zweite Rückhalte-Führungsnuten 46, die auch parallel zueinander und zu den Rückhalte-Führungsnuten 18 verlaufen. Die Rückhalte-Führungsnuten 18 bilden erste Rückhalte-Führungsnuten 18. Die zweiten Rückhalte-Führungsnuten 46 sind einander gegenüberliegend angeordnet. Sie sind in einander gegensinnigen Richtungen nach seitlich außen offen. Die zweiten Rückhalte-Führungsnuten 46 sind benachbart zu den ersten Rückhalte-Führungsnuten 18 angeordnet. Der Abstand zwischen einer ersten Rückhalte-Führungsnut 18 und der benachbart angeordneten zweiten Rückhalte-Führungsnut 46 entspricht im Wesentlichen dem Abstand der ersten Rückhaltenase 39 zu der benachbart angeordneten zweiten Rückhaltenase 44.

Nachfolgend wird das Dosiersystem gemäß der zweiten Ausführungsform im Einsatz beschrieben. Dabei wird zunächst von einem Zustand ausgegangen, bei dem sich das Auszieh-Begrenzungselement 5a in seiner radial äußeren, unwirksamen Position befindet. Dieser Zustand ist in Fig. 10 veranschaulicht.

Bei diesem Zustand steht das zweite Formschlussmittel 35a außer Eingriff mit den ersten Formschlussmitteln 20, sodass das Aufzieh-Begrenzungselement 5a längs der Längsachse 1 gegenüber dem Dosierbehälter 3a verschiebbar ist. Die axiale Verschiebung des Aufzieh-Begrenzungselements 5a gegenüber dem Dosierbehälter 3a ist durch die ersten Rückhaltenasen 39 geführt, die von außen seitlich in die zweiten Rückhalte-Führungsnuten 46 eingreifen. Die an dem Einstellansatz 10a im Bereich der zweiten Rückhalte-Führungsnuten 46 angreifenden bzw. diesem zugewandten Rückhalteflächen 40 verhindern bzw. erschweren dabei ein Entfernen des Aufzieh-Begrenzungselements 5a in radialer Richtung nach außen von dem Dosierbehälter 3a. Die zweiten Rückhaltenasen 44 sind hier im Wesentlichen ohne Führungs- bzw. Rückhaltefunktion.

In einem Bereich zwischen den freien Enden 41 und den ersten Rückhaltenasen 39 verlaufen die U-Schenkel 32a im Wesentlichen beabstandet zu dem Dosierbehälter 3a.

Beim axialen Verschieben des Aufzieh-Begrenzungselements 5a zum Einstellen des Dosiervolumens greift das zweite Formschlussmittel 35a kurzfristig etwas in die ersten Formschlussmittel 20 ein, was zu einer akustischen Rückmeldung in Form eines Rattergeräusches führt.

Das Aufzieh-Begrenzungselement 5a wird dann in einer gewünschten Begrenzungsposition axial an dem Einstellansatz 10a festgelegt. Das axiale Festlegen des Aufzieh-Begrenzungselements 5a gegenüber dem Dosierbehälter 3a wird erreicht, indem auf den U-Boden 33 derart von außen eine manuelle Kraft in Richtung auf die Längsachse 1 ausgeübt wird, dass sich der U-Boden 33 der Außenseite 19 des Einstellansatzes 10a nähert. Die radial innere Position des Aufzieh-Begrenzungselements 5a ist erreicht, wenn die Innenseite 34 des U-Bodens 33 an der Außenseite 19 des Einstellansatzes 10a zumindest bereichsweise anliegt bzw. unmittelbar benachbart zu dieser angeordnet ist und so das zweite Formschlussmittel 35a mit dem entsprechenden benachbarten ersten Formschlussmittel 20 in einer wieder lösbaren Steckverbindung steht.

Das zweite Formschlussmittel 35a greift in der radial inneren Position des Aufzieh-Begrenzungselements 5a von außen durch das benachbarte erste Formschlussmittel 20 in die Führungsaufnahme 17 ein und steht nach innen gegenüber dem ersten Formschlussmittel 20 über. Es bildet dabei einen Gegenanschlag für den Anschlag 6. Das Aufzieh-Begrenzungselement 5a ist durch die formschlüssige Verbindung der Formschlussmittel 20, 35 miteinander axial an dem Dosierbehälter 3a festgelegt.

Beim Überführen des Aufzieh-Begrenzungselements 5a von der radial äußeren in die radial innere Position gelangen die ersten Rückhaltenasen 39 außer Eingriff mit den zweiten Rückhalte-Führungsnuten 46.

Die ersten Rückhaltenasen 39 stehen nun in der radial inneren Position des Aufzieh-Begrenzungselements 5a mit den ersten Rückhalte-Führungsnuten 18 rastend in Eingriff, während die zweiten Rückhaltenasen 44 mit den zweiten Rückhalte-Führungsnuten 46 rastend in Eingriff stehen. Die dem U-Boden 33 zugewandten bzw. an dem Einstellansatz 10a anliegenden Rückhalteflächen 40 und 46 der ersten bzw. zweiten Rückhaltenasen 39, 44 verhindern bzw. erschweren ein Bewegen des Aufzieh-Begrenzungselements 5a nach radial außen. Es ist von Vorteil, wenn die ersten Rückhaltenasen 39 für die radiale Fixierung des Aufzieh-Bregrenzungselements 5a zuständig sind.

Das Aufzieh-Begrenzungselement 5a ist nun axial und radial sowie in Umfangsrichtung um die Längsachse 1 an dem Dosierbehälter 3a fixiert.

Günstigerweise ist der Abstand zwischen der ersten Rückhalte-Führungsnut 18 und der benachbart angeordneten zweiten Rüchhalte-Führungsnut 46 kleiner als der Abstand der zweiten Rückhalte-Führungsnut 46 zu den ersten Formschlussmitteln 20 bzw. zu dem Scheitelbereich des Einstellansatzes 10a. Dies ermöglicht eine besonders einfache Überführung des Aufzieh-Begrenzungselements 5a in eine radial äußere Reinigungsstellung.

## Patentansprüche

1. Dosiersystem zur Einstellung eines Dosiervolumens eines fließfähigen Mediums, umfassend
a) eine Längsachse (1),
b) einen einteiligen Dosierbehälter (3; 3a) zur Aufnahme des Mediums,
i) wobei der Dosierbehälter (3; 3a) mindestens eine Kommunizierungsöffnung (7) zur Kommunizierung mit einer Umgebung aufweist,
c) einen sich längs der Längsachse (1) erstreckenden Dosier-Kolbenkörper (4), der in dem Dosierbehälter (3; 3a) abgedichtet verlagerbar ist zwischen
i) einer Ausgangsposition, in der der Dosier-Kolbenkörper (4) ganz in den Dosierbehälter (3; 3a) eingeschoben ist, und
ii) einer Dosierposition, in der der Dosier-Kolbenkörper (4) bis zu einer der gewünschten Dosiermenge des Mediums entsprechenden Wegstrecke aus dem Dosierbehälter (3; 3a) herausgezogen ist,
d) ein Aufzieh-Begrenzungselement (5; 5a) zum Begrenzen eines Aufziehwegs des Dosier-Kolbenkörpers (4) in der Dosierposition, wobei das Aufzieh-Begrenzungselement (5; 5a) in einer von mehreren Begrenzungspositionen relativ zu der Längsachse (1) an dem Dosierbehälter (3; 3a) axial festlegbar ist,
i) wobei das Aufzieh-Begrenzungselement (5; 5a) radial zu der Längsachse (1) zwischen einer inneren, axial an dem Dosierbehälter (3; 3a) festgelegten Begrenzungsposition und einer äußeren Position gegenüber dem Dosierbehälter (3; 3a) verlagerbar ist, und
e) einen Anschlag (6) zur Anlage an dem Aufzieh-Begrenzungselement (5; 5a) zum Begrenzen des Aufziehwegs des Dosier-Kolbenkörpers (4) in der Dosierposition,
i) wobei der Anschlag (6) axial fest mit dem Dosier-Kolbenkörper (4) verbunden ist,
ii) wobei das Aufzieh-Begrenzungselement (5; 5a) so ausgeführt ist, dass es nur dann mit dem Anschlag (6) in Kontakt bringbar ist, wenn es sich in seiner radial inneren Position befindet.

2. Dosiersystem nach Anspruch 1, **dadurch gekennzeichnet, dass** das Aufzieh-Begrenzungselement (5; 5a) außen an dem Dosierbehälter (3; 3a) zwischen mindestens zwei Dosierpositionen in Richtung der Längsachse (1) axial verlagerbar ist, wenn es sich in seiner radial äußeren Position befindet.

3. Dosiersystem nach Anspruch 1 oder 2, **dadurch gekennzeichnet, dass** sich das in der radial äußeren Position befindende Aufzieh-Begrenzungselement (5; 5a) bei einer axialen Relativbewegung zu dem Dosierbehälter (3; 3a) eine Rückmeldung, vorzugsweise eine akustische und/oder taktile Rückmeldung, in Bezug auf die Begrenzungspositionen gibt.

4. Dosiersystem nach einem der vorherigen Ansprüche, **gekennzeichnet durch** eine zwischen dem Aufzieh-Begrenzungselement (5; 5a) und dem Dosierbehälter (3; 3a) wirkende Rückhalteeinrichtung (20, 35, 18, 39; 44, 46), die die radiale Verlagerung des Aufzieh-Begrenzungselements (5; 5a) nach außen begrenzt oder erschwert.

5. Dosiersystem nach einem der vorherigen Ansprüche, **dadurch gekennzeichnet, dass** das Aufzieh-Begrenzungselement (5; 5a) in der gewünschten Dosierposition mit dem Dosierbehälter (3; 3a) über eine Rastverbindung (20, 35, 18, 39; 44, 46) verrastbar ist.

6. Dosiersystem nach einem der vorherigen Ansprüche, **dadurch gekennzeichnet, dass** das Aufzieh-Begrenzungselement (5; 5a) einen äußeren Umfangsabschnitt des Dosierbehälters (3; 3a) umgreift.

7. Dosiersystem nach einem der vorherigen Ansprüche, **dadurch gekennzeichnet, dass** das Aufzieh-Begrenzungselement (5; 5a) eine Markierung (42) aufweist, die zusammen mit einer Dosierskala (11) an dem Dosierbehälter (3; 3a) eine einzustellende Dosiermenge des Mediums anzeigt.

8. Dosiersystem nach einem der vorherigen Ansprüche, **dadurch gekennzeichnet, dass** der Anschlag (6) benachbart zu einem Kolbenboden (23) des Dosier-Kolbenkörpers (4) zum Ausstoßen des Mediums angeordnet ist.

9. Dosiersystem nach einem der vorherigen Ansprüche, **dadurch gekennzeichnet, dass** der Anschlag (6) einstückig mit dem Dosier-Kolbenkörper (4) verbunden ist.

10. Dosiersystem nach einem der vorherigen Ansprüche, **dadurch gekennzeichnet, dass** der Anschlag (6) fest mit einem parallel zu dem Dosier-Kolbenkörper (4) verlaufenden Dosier-Kolbenausleger (30) verbunden ist, wobei der Dosier-Kolbenausleger (30) axial fest mit einem Grundkörper (26) des Dosier-Kolbenkörpers (4) verbunden ist.

11. Dosiersystem nach Anspruch 10, **dadurch gekennzeichnet, dass** der Dosier-Kolbenausleger (30) in einer Führungsaufnahme (17) des Dosierbehälters (3; 3a) verschiebbar geführt ist.

12. Dosiersystem nach Anspruch 10 oder 11, **dadurch gekennzeichnet, dass** der Dosier-Kolbenkörper (4) einen axialen Endbereich (28) aufweist, wobei der Dosier-Kolbenausleger (30) von dem axialen Endbereich (28) ausgeht und benachbart zu dem Grundkörper (26) des Dosier-Kolbenkörpers (4) verläuft.

13. Dosiersystem nach Anspruch 11, **dadurch gekennzeichnet, dass** der Dosierbehälter (3; 3a) einen Aufnahmeraum (8) zur Aufnahme des Mediums aufweist, wobei der Aufnahmeraum (8) räumlich getrennt zu der Führungsaufnahme (17) angeordnet ist.

14. Dosiersystem nach einem der vorherigen Ansprüche, **dadurch gekennzeichnet, dass** das Aufzieh-Begrenzungselement (5; 5a) in seiner radial äußeren Position außer Stande ist, mit dem Anschlag (6) wechselzuwirken.
